(19) Europäisches Patentamt
European Patent Office
Office européen des brevets

(11) **EP 3 139 156 A1**

(12) **EUROPEAN PATENT APPLICATION**

(43) Date of publication:
08.03.2017 Bulletin 2017/10

(21) Application number: 15183946.1

(22) Date of filing: 04.09.2015

(51) Int Cl.:
*G01N 23/04* (2006.01)      *A61B 6/00* (2006.01)
*G21K 1/00* (2006.01)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA

(71) Applicant: **Paul Scherrer Institut**
**5232 Villigen (CH)**

(72) Inventors:
• STAMPANONI, Marco
  5304 Endingen (CH)
• KAGIAS, Matias
  8047 Zürich (CH)
• WANG, Zhentian
  5200 Brugg (CH)

(74) Representative: **Fischer, Michael**
**Siemens AG**
**Postfach 22 16 34**
**80506 München (DE)**

(54) **DUAL PHASE GRATING INTERFEROMETER FOR X-RAY PHASE CONTRAST IMAGING**

(57)    Since the very first experiments with phase-contrast imaging at synchrotrons, X-ray scientists were quite excited by the potential of this novel approach, as the "holy-grail" of boosting the contrast of soft and radiation sensitive materials under dose-control seemed to be finally at reach. The features of gratings-based interferometry (GI) are well suited for transferring this exciting technology from the exclusive synchrotron's community to a much wider basin of potential users. Particularly for medical applications, the relation between image contrast and dose has triggered tremendous efforts in the development of novel imaging devices. Such systems essentially operate near to the photon-starvation limit to cope with the fundamental dilemma of providing sufficient diagnostic sensitivity and sensibility at an acceptable, as low as reasonably achievable (ALARA) risk for the patient. If a new imaging modality were to be implemented in a clinical environment, it is needless to say that it has to be compliant with the very strict regulatory directives.

The present invention proposes a system based exclusively on X-ray phase shifting components, i.e. without the use of an absorption grating, or a mask or a high-resolution detector. The novel approach is applicable at all imaging relevant energies and can be easily scalable to large field of views. The invention solves in one shot most the major limitations so far which were preventing a broad dissemination of phase contrast X-ray imaging on conventional sources.

FIG 2A

**(Cont. next page)**

# FIG 2B

Virtual structured
illumination (G1')
generated by G1

Interference
pattern (G2') on
the detector plane

# FIG 2C

**Description**

The present invention relates to an arrangement for x-rays, in particular hard x-rays, for obtaining quantitative x-ray images from a sample.

Conventional grating interferometry constitutes a very promising technique for commercial X-ray phase-contrast applications, since it works with traditional X-ray tubes, is mechanically robust and has modest requirements for mono-chromaticity and spatial coherence. In the last few years, several exciting applications of this technique have been reported, ranging from material inspection to medical imaging. To carry out the transition of grating interferometry from the synchrotrons and physics laboratory to the commercial setting (medical, homeland security, nondestructive testing or similar), the technology has to be further developed to cover a large field of view (FOV), to allow operation in a broad energy range and to be dose efficient.

These issues quickly became serious problems, as in all the suggestions presented so far either absorption gratings of very large aspect ratio were needed or the usage of high-resolution detectors was required -- which are notoriously incompatible with large areas -- or the costs in terms of additional radiation deposited in the sample due to the absorption gratings were inacceptable. Although the FOV issue can be partially mitigated using a slit-scanning approach, the requirement on high aspect ratio absorption grating for high-energy applications remains a huge fabrication challenge. An even more critical and intrinsic issue of grating interferometry is the dose efficiency due to the usage of absorption grating (the so-called analyzer grating that is in front of the detector). Though the absorption grating decouples the system sensitivity from the system spatial resolution which in fact is the key to the success of the grating interferometer, its existence reduces the system's dose efficiency and flux efficiency by half which severely limits the applications of grating interferometry, especially in medical imaging.

An interferometric method without the absorption grating will naturally solve the dose and flux efficiency issue, and avoid the fabrication challenge, ideally with fast data acquisition, will become desirable for clinical and industrial applications.

The present invention provides an arrangement for x-rays, in particular hard x-rays, for obtaining quantitative x-ray images from a sample; comprising:

> a. an X-ray source (x-ray), preferably a standard polychromatic X-ray source;
> b. two phase-shift gratings (G1, G2);
> c. a position-sensitive detector (PSD) with spatially modulated detection sensitivity having a number of individual pixels;
> d. means for recording the images of the detector (PSD) ;
> e. means for evaluating the intensities for each pixel in a series of images in order to identify the characteristic of the object for each individual pixel as an absorption dominated pixel and/or a differential phase contrast dominated pixel and/or an x-ray scattering dominated pixel;

> f. an optional absorption grating or mask (GO) in front of, or embedded into the X-ray source.

The present invention represents a crucial step towards X-ray absorption-grating-less interferometry. The present invention generates a large period interference pattern (i.e. removing the need of a high-resolution detector) by using only phase gratings. One phase grating is used to create a secondary source, and another phase grating is to form an inference pattern under the illumination of the secondary source. This new phase-phase gratings solution instead of the phase-absorption or absorption-absorption suggestions will allow to exploit essentially 100% of the photons (as the attenuation in the phase gratings can be considered as negligible) therefore solving the limitation of all interferometers presented so far, which are cutting half of the photons (or more) after the sample. In addition, and probably the most striking point of this invention, since the phase shift varies linearly with the energy (in comparison to the $E^{-3}$ dependency of the attenuation signal) it results that phase-shifting gratings for high energies > 60 keV are easily achievable with state-of-the-art fabrication methods. In general, the fabrication of a phase grating is much easier than absorption grating, and the quality control can be done better as well. These points remove the most significant burden from a wide application of grating interferometry.

The present invention may have preferred embodiments which can be generated by one or a suitable combination of some or all the features listed below:

- G1 and G2 are line (1D) or 2D array (i.e. chessboard) phase gratings, that is the periodic structures generating a considerable X-ray phase shift difference, the latter preferably of $\pi$ or odd multiples thereof (denoted as $\pi$ shift), or $\pi/2$ or $\pi/2+N\times2\times\pi$ (denoted as $\pi/2$ shift), where N is a integer number;

- the phase G1 and G2 are both $\pi$ shift gratings, or both $\pi/2$ shift gratings, or one of them is $\pi$ shift grating and the other is $\pi/2$ shift grating;

- the phase grating is made by deep etching into silicon, a polymer or similar material, preferable for low energy X-ray photons; or deposit heavy metal into gaps of low-absorbing structure or grow heavy metal on low-absorbing substrate and as use the metal as the phase shift material, preferably for high energy X-ray photons;

- the first grating G1 creates a periodic interference pattern with pitch $p_1'$ at a known distance (Talbot effect) downstream and acts as structured illumination onto the second grating G2, which further creates a periodic interference pattern with pitch $p_2'$ at the detector plane. $p_1'$ and $p_2'$ match the radius of curvature of an incident wavefront by the relation

$$p_1' = \frac{1}{\eta} p_1 \frac{d_1 + l_1}{l_1}, \qquad p_2' = \frac{1}{\eta} p_2 \frac{d_2 + l_2}{l_2} \qquad \text{and}$$

$\frac{p_1'}{p_2'} = \frac{d_2}{l_2}$ , where $p_1$ and $p_2$ are the pitch of G1 and G2, respectively, $l_1$ is the distance between the source (or G0 if G0 is used) to G1 distance, $d_1$ is the distance between G1 and the created structured illumination, $d_2$ is the distance between the structured illumination and G2, $l_2$ is the distance between G2 and the detector, $\eta = 1$ for $\pi$ shift grating while $\eta = 2$ for $\pi/2$ shift grating;

- the phase shift of G1 and G2 and the distances between the source (or G0 if G0 is used), G1, G2 and the detector are adapted to a photon energy corresponding to an emission line of the X-ray generator used as the source;

- a mechanism is comprised to vary the angular orientation, around the optical axis, of the G1 and G2 with respect to each other, so that the orientation of the interference pattern on the detector plane is tuned in order to obtain sample information along a specific direction;

- a mechanism is comprised to vary the distance between G1 and G2, or that between source (or G0 if G0 is used) and G1, or that between G2 and the detector, along the optical axis, so that the pitch of the interference pattern on the detector plane is tuned in order to obtain sample image with desired spatial resolution, or desired sensitivity, or work with detectors with certain specific pixel size;

- a mechanism is comprised to vary the distance between G1 and G2, or that between source (or G0 if G0 is used) and G1, or that between G2 and the detector, along the optical axis, so that the duty cycle of the interference pattern on the detector plane is tuned in order to obtain interference pattern with specific duty cycle;

- a mechanism is comprised to incline the phase grating G1 and G2 together or with respect to each other along the optical axis, so that the phase shifts of G1 and G2 is tuned to be $\pi$ shift or $\pi/2$ shift to match a tunable photon energy therefore the arrangement can work at different photon energy with the same phase shift gratings;

- the phase gratings G1 and G2 are identical, featuring a symmetric system design, that is to be said, $l_1 = l_2$ and $d_1 = d_2$;

- the phase gratings G1 and G2 have different pitches, featuring an asymmetric system design;

- G0 is not used but the X-ray source comprises 1D or 2D array of individual sources that may be mutually incoherent and whose lateral separation

$$p_0 = p_1' \times \frac{l_1}{d_1} \quad \text{or integer multiples thereof;}$$

- the array of X-ray source is generated by using an anode that structured topographically or assembled in a mosaic manner from the same or different materials;

- a mechanism is comprised to place a sample (S) to be investigated between the X-ray source (or G0 if G0 is used) and G1, or between G1 and G2, or between G2 and the detector;

- an analysis procedure is implemented for obtain the absorption, differential phase contrast and scattering contrast of the sample that comprises the steps of recording two intensity images of the interference pattern (with sample and without sample) on the detector, and analyzing the local distortions of the two interference patterns by either spatial Fourier analysis or spatial correlation analysis, and then retaining the three contrast of the sample;

- a mechanism is comprised to lateral move one of the gratings (G0, G1 and G2) with a fraction of its pitch and records a serial of images on the detector, and an analysis procedure is implemented for this phase-stepping data scan that comprises the steps of calculating, for each element of the detector, the Fourier transform of the intensity curve measured in the element, and then retaining the Fourier components as signals for further processing;

- means for rotating the sample relatively to the remaining components to perform data collection for a tomographic scan.

- the phase grating G1 and G2 are fabricated on the two sides of the same wafer, with grating structures either parallel to each other or with a predefined angle;

- G1 is a 2D chessboard/mesh-type grating, and G2 is a 2D array grating, with each element of the array to be a circular grating; and

- G1 and G2 are absorption gratings.

Preferred embodiments of the present invention are described hereinafter in detail with reference to the attached drawings which depict in:

Fig. 1 schematically a conventional Talbot-Lau grating interferometer;

Fig. 2 a sketch of the dual phase gratings interferometer and its working mechanism; (a) 3D sketch of the proposed interferometer; (b) Top view of the proposed interferometer; (c) Side view of the proposed interferometer;

Fig. 3. experimental results of the proposed interferometer;

Fig. 4 fringe tuning by changing the inter-grating (G1-G2) distance;

Fig. 5 imaging a plastic tube using the arrangement shown in Fig. 2; (a) The transmission image; (b) The differential phase image; and

Fig. 6 tilting G1 and G2 together along the optical axis to work with different design energy.

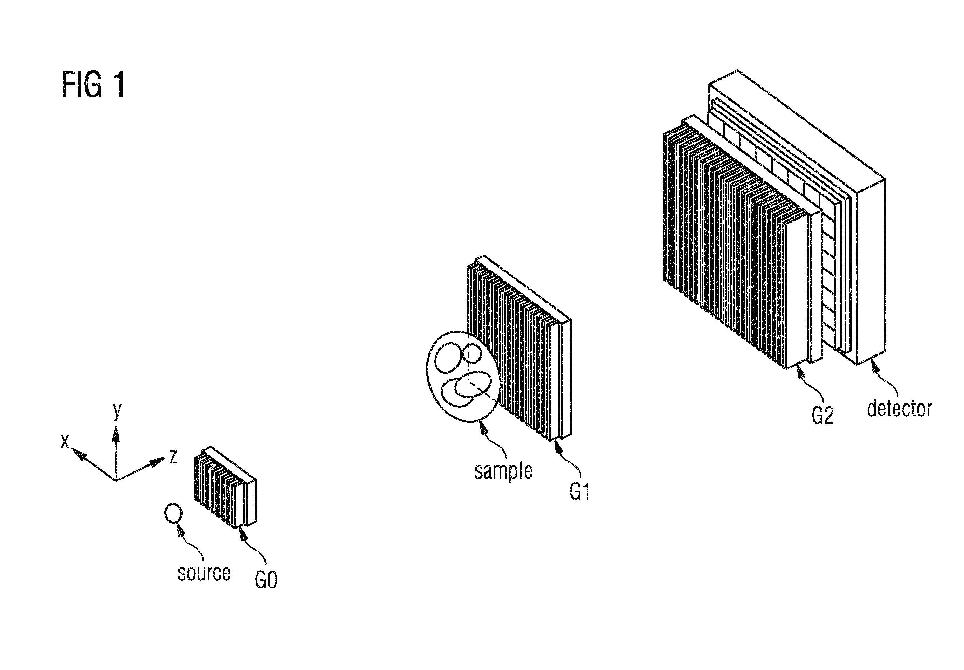

A conventional Talbot-Lau grating interferometer is shown in Fig. 1, where G0, G1 and G2 are the source, phase and absorption gratings, respectively. The use of G0 is optional, depending on the spatial coherence properties of the X-ray source.

The conventional grating interferometer decouples the system sensitivity and the detector spatial resolution by introducing an acquisition protocol named phase stepping. In the phase stepping procedure, one of the gratings is laterally translated step-by-step, perpendicular to the grating lines, and a series of images are taken for each step. For each element of the detector, a sinusoid intensity curve is then recorded. The absorption contrast (AC), differential phase contrast (DPC) and scattering contrast (SC) of the sample are calculated by comparing the intensity curve without the sample and that with the sample. Defining for each pixel on the detector the mean, phase and visibility of the intensity curve with sample as $I_S, \phi_S, V_S$, and without sample as $I_b, \phi_b, V_b$, yields:

$$AC = -\log\left(\frac{I_S}{I_b}\right)$$

$$(1)$$

$$DPC = \phi_s - \phi_b$$

$$(2)$$

$$SC = -log\left(\frac{V_S}{V_b}\right)$$

$$(3)$$

One disadvantage of the conventional grating interferometer is that the phase stepping procedure is quite time-consuming and therefore renders applications that need fast scanning or time-resolution not possible. Furthermore, it requires additional mechanical movement of the optical components, which poses higher requirements on the system stability and design. Both issues are undesired for clinical and industrial applications.

To avoid the time-consuming phase stepping scan, a few single-shot X-ray phase-contrast imaging (PCI) methods using gratings have been proposed. Like conventional X-ray imaging modalities, single-shot PCI methods only take two images: one reference image without sample and one sample image. In order to obtain three different contrasts in a single shot, these methods require that the detector has sufficient resolution to resolve the fringe pattern generated by the gratings, via either interference effect or simple projection, directly. The sensitivity of such methods is largely inverse proportional to the pitch of the fringe on the detector, therefore is indirectly limited by the detector pixel size.

Nevertheless, the existing single-shot methods still use absorption gratings to generate the fringe pattern, either use it together with a phase grating to create a large period Moire fringe, or use large pitch absorption grating or grid to create a projected fringe directly.

One possible solution without using an absorption grating is the inverse geometry grating interferometer, however this method is dictated by the discrete property of the Talbot(-Lau) effect, that being said, the system total length, the pitches of the gratings and the photon energy are strongly coupled and can't be tuned continuously and arbitrarily. For the energy range (8-30 keV) used in literatures, the inverse geometry interferometer results in either impractical, very long system length (>> 2m) or very small pitch G0 (<< 2$\mu$m) that is not possible to fabricate at this stage. For clinical and industrial applications, the system length has to be within a reasonable range to utilize the limited flux of the X-ray tube (since the flux is inverse proportional to the $R^2$, as $R$ to be the source to detector distance). A compact system is usually desired due to the higher flux efficiency, therefore the inverse geometry approach is not practical.

Alternative single-shot DPC methods using speckles have also been proposed. However these methods pose harsh requirements on the coherence of the X-ray source, therefore are limited to either synchrotrons or micro-focal X-ray source with very long setup (3m).

The present invention is essentially a single-shot DPC method, overcoming the aforementioned limitations of existing methods. The present invention consists of an X-ray arrangement, which measures absorption, phase and scattering signals from a sample with the usage of a phase-phase grating combination. It addresses the following difficulties:

- It exploits all photons transmitted through the sample to generate contrast;
- It can be implemented at energies varying over a very broad range;
- It has a tunable working energy;
- It can cover large field of view;
- It works in symmetrical, asymmetrical and magnified geometry; and
- Is compatible with single-shot imaging.

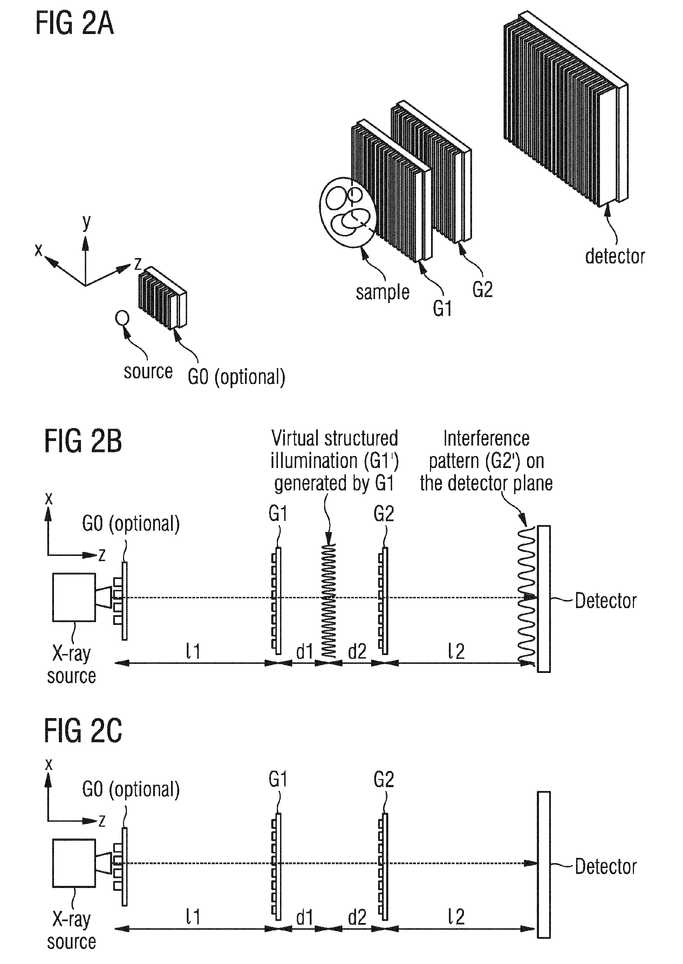

The idea behind the present invention, dubbed as dual phase gratings interferometer, is shown in Fig. 2. Since only phase gratings are used, the dose and flux efficiency of the present invention is much better than existing methods. Fig. 2 shows a sketch of the dual phase gratings interferometer and its working mechanism; (a) 3D sketch of the proposed interferometer; (b) Top view of the proposed interferometer; (c) Side view of the proposed interferometer.

In the proposed invention, a first phase grating G1 creates a periodic interference pattern at a known distance downstream. This intensity distribution can be considered as a secondary source with structured illumination, which is used to generate a large-period interference pattern on the detector with the usage of a second phase grating G2. In principle, the optional G0, G1 and the virtual fringe G1' constitute a conventional Talbot (-Lau) interferometer, and G1', G2 and the interference pattern on the detector G2' constitute another Talbot (-Lau) interferometer. Depending on the pitches of the gratings, such a device can be "symmetric" (i.e. G1 and G2 are identical for fabrication benefit, resulting in equal distance between the source-to-G1 and the G2-to-detector) or asymmetrical (i. e. arbitrary configuration for flexible system design).

Like conventional Talbot-(Lau) grating interferometer, G0 is only necessary when an extended X-ray source is used in order to produce sufficient coherence. However, unlike an inverse geometry grating interferometer whose G0 may have much smaller pitch than G1, the pitch of G0 in the proposed interferometer is always equal to or larger then the pitch of G1, therefore won't pose any fabrication difficulty.

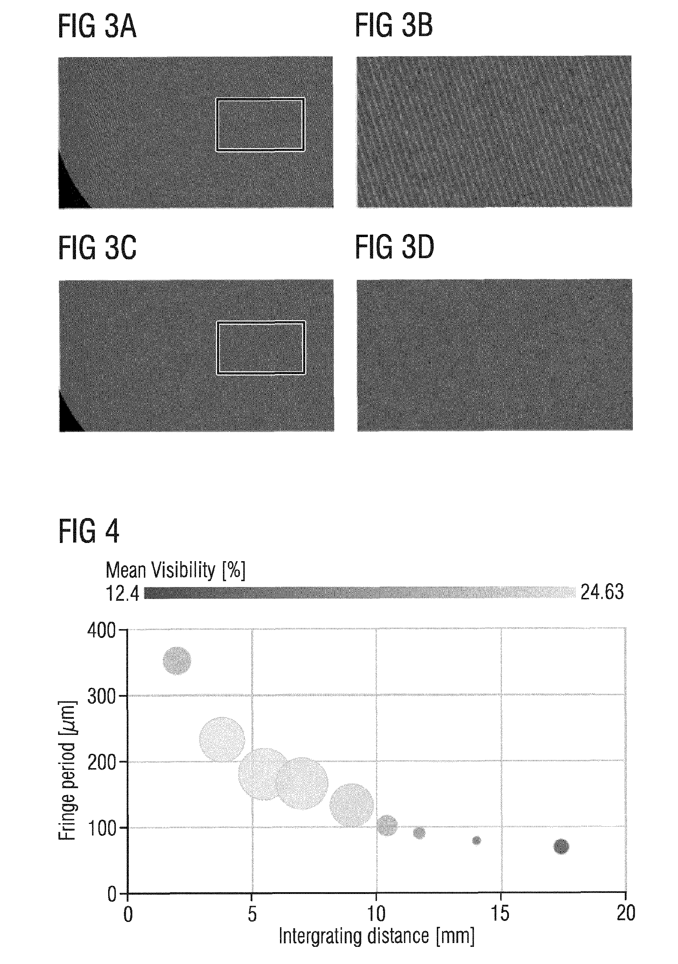

An example of the interference fringes generated with the proposed interferometer is shown in Fig. 3. The experiment was carried on with a micro-focal X-ray tube (operated at 50kVp) and two identical phase gratings ($\pi$ shift @ 17KeV) with the pitch of $1.2 \mu m$. The interference pattern on the detector plane only exists when the second phase grating G2 is introduced.

Fig. 3 shows the experimental results of the proposed interferometer. (a) and (c) are the images acquired on the detector with and without the second grating G2, respectively. Once G2 is removed from the beam path, the interference pattern disappeared. (b) and (d) are the corresponding ROI images of the rectangular in (a) and (c).

The proposed invention explores the same physical effect (i.e. Talbot effect) as the conventional Talbot-(Lau) grating interferometer, therefore it inherits some similar properties, for instance, the relationship between the pitches of the gratings and the distances ($l_1$ and $d_1$, $l_2$ and $d_2$) are following the same formulas, but due to the unique design, the proposed approach provides much more flexibility in designing a compact system.

For example, consider a single-shot DPC system that requires a design energy of 25KeV and a total system length of 1m, and has a detector that can resolve interference pattern with $100 \mu m$ pitch. An inverse geometry system will require an absorption grating G0 with less than $1 \mu m$ pitch and the G0-G1 distance will be less than 1cm which results in useless FOV. Using the proposed dual phase grating approach with a symmetric design, the pitch of the optional G0 is $100 \mu m$ and the pitches of the two phase gratings will be $1 \mu m$. Compared to the inverse geometry case, it's obviously much easier to fabricate $1 \mu m$ phase grating than absorption grating.

The proposed invention works well with monochromatic beam, however when working with polychromatic beam, it provides more flexibilities to cope with different requirements due to the broad energy spectrum acceptance of the Talbot-(Lau) interferometer. In one implementation, the distance between G1 and G2 can be tuned, resulting in variable pitch of interference fringe on the detector. This feature can be used to tune the system's sensitivity or to match a specific detector pixel size, for instance, using different binning of the detector. An experimental result is shown in Fig. 4.

Fig. 4 shows a fringe tuning by changing the inter-grating (G1-G2) distance. The designed distance between G1 and G2 distance is 5mm in this case. Highest visibility is obtained at the designed position. While tuning the inter-grating distance, the pitch of the fringe is tuned as well.

The orientation of the interference fringe on the detector can be easily changed by rotating one of G1 and G2, or G1 and G2 together. In the meantime, the pitch of the fringe will stay constant. This allows one to obtain multiple directional DPC and scattering signals of the sample with constant sensitivity.

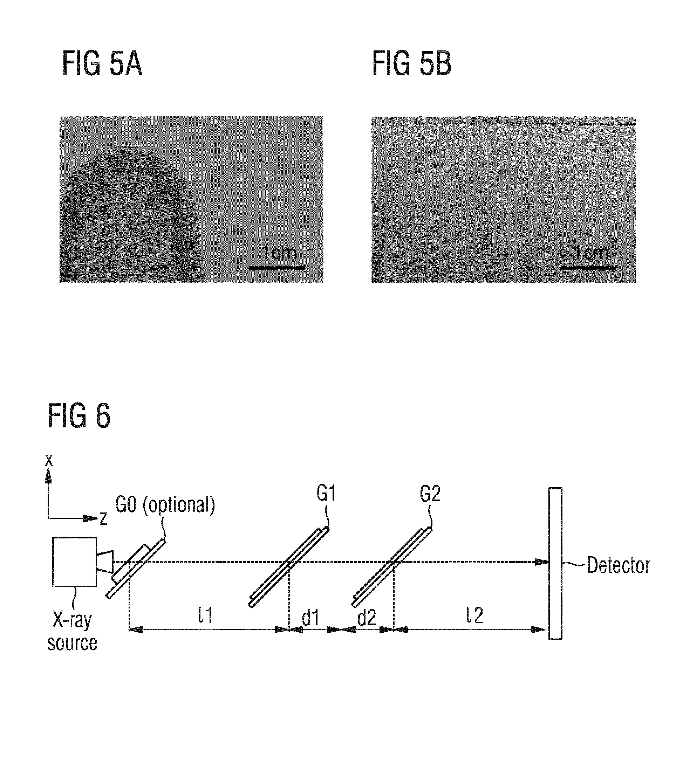

The sample can be placed between the X-ray source (or G0 if G0 is used) and G1, or between G1 and G2, or between G2 and the detector. To achieve the highest sensitivity, it's preferable to place the sample close to G1 or G2. An imaging result is shown in Fig. 5. In detail, Fig. 5 shows an imaging a plastic tube using the proposed method. (a) The transmission image; (b) The differential phase image.

For the proposed interferometer, the inter-grating distance (G1-G2) can be very small. Eventually they can be fabricated on the two sides of the same wafer (i.e.

etching on both sides of a Silicon wafer), therefore G1 and G2 become one optical component. This will significantly reduce the complexity of the setup.

Due to the nature of phase grating, the proposed interferometer can be easily tuned to work perfectly with different design energies. By tilting G1 and G2 together along the optical axis (see Fig. 6), the effective height of the phase-shifting structure is increased, and the system naturally works with higher design energy. Fig. 6 shows the tilting of G1 and G2 together along the optical axis to work with different design energy.

Another advantage of using purely phase gratings is that it's easier to work with high-energy photons. High Z material (i.e. Au) can be used as phase shift material in high-energy situations. The required aspect ratio of the grating is much lower for phase grating compared to absorption grating, resulting in significant fabrication benefits. For example, for 100KeV photon, absorption grating with 10% transmission will require of Gold structure of at least 230$\mu$m, while a $\pi$-shift Gold grating will only need 19.8$\mu$m.

## Claims

1. An arrangement for x-rays, in particular hard x-rays, for obtaining quantitative x-ray images from a sample; including:

   a) an X-ray source (x-ray), preferably a standard polychromatic X-ray source;
   b) two phase-shift gratings (G1, G2);
   c) a position-sensitive detector (PSD) with spatially modulated detection sensitivity having a number of individual pixels;
   d) means for recording the images of the detector (PSD); e)
   e) means for evaluating the intensities for each pixel in a series of images in order to identify the characteristic of the object for each individual pixel as an absorption dominated pixel and/or a differential phase contrast dominated pixel and/or an x-ray scattering dominated pixel;
   f) an optional absorption grating or mask (G0) in front of, or embedded into the X-ray source.

2. The arrangement according to claim 1, wherein G1 and G2 are line (1D) or 2D array (i.e. chessboard) phase gratings, that is the periodic structures generating a considerable X-ray phase shift difference, the latter preferably of $\pi$ or odd multiples thereof (denoted as $\pi$ shift), or $\pi/2$ or $\pi/2$+N$\times$2$\times\pi$ (denoted as $\pi/2$ shift), where N is a integer number.

3. The arrangement according to claim 1 or 2, wherein the phase G1 and G2 are both $\pi$ shift gratings, or both $\pi/2$ shift gratings, or one of them is $\pi$ shift grating and the other is $\pi/2$ shift grating.

4. The arrangement according to claim 1-3, wherein the phase grating is made by deep etching into silicon, a polymer or similar material, preferable for low energy X-ray photons; or deposit heavy metal into gaps of low-absorbing structure or grow heavy metal on low-absorbing substrate and as use the metal as the phase shift material, preferably for high energy X-ray photons.

5. The arrangement according to claim 1-4, wherein the first grating G1 creates a periodic interference pattern with pitch $p'_1$ at a known distance (Talbot effect) downstream and acts as structured illumination onto the second grating G2, which further creates a periodic interference pattern with pitch $p'_2$ at the detector plane. $p'_1$ and $p'_2$ match the radius of curvature of an incident wavefront by the relation

$$p'_1 = \frac{1}{\eta} p_1 \frac{d_1 + l_1}{l_1}, \qquad p'_2 = \frac{1}{\eta} p_2 \frac{d_2 + l_2}{l_2} \quad \text{and}$$

$$\frac{p'_1}{p'_2} = \frac{d_2}{l_2} \;, \text{ where } p_1 \text{ and } p_2 \text{ are the pitch of G1}$$

and G2, respectively, $l_1$ is the distance between the source (or G0 if G0 is used) to G1 distance, $d_1$ is the distance between G1 and the created structured illumination, $d_2$ is the distance between the structured illumination and G2, $l_2$ is the distance between G2 and the detector, $\eta$ = 1 for $\pi$ shift grating while $\eta$ =2 for $\pi/2$ shift grating.

6. The arrangement according to claim 1-5, wherein the phase shift of G1 and G2 and the distances between the source (or G0 if G0 is used), G1, G2 and the detector are adapted to a photon energy corresponding to an emission line of the X-ray generator used as the source.

7. The arrangement according to any of the preceding claims, wherein a mechanism is comprised to vary the angular orientation, around the optical axis, of the G1 and G2 with respect to each other, so that the orientation of the interference pattern on the detector plane is tuned in order to obtain sample information along a specific direction.

8. The arrangement according to any of the preceding claims, wherein a mechanism is comprised to vary the distance between G1 and G2, or that between source (or G0 if G0 is used) and G1, or that between G2 and the detector, along the optical axis, so that the pitch of the interference pattern on the detector plane is tuned in order to obtain sample image with desired spatial resolution, or desired sensitivity, or work with detectors with certain specific pixel size.

**9.** The arrangement according to any of the preceding claims, wherein a mechanism is comprised to vary the distance between G1 and G2, or that between source (or G0 if G0 is used) and G1, or that between G2 and the detector, along the optical axis, so that the duty cycle of the interference pattern on the detector plane is tuned in order to obtain interference pattern with specific duty cycle.

**10.** An arrangement according to any of the preceding claims, wherein a mechanism is comprised to incline the phase grating G1 and G2 together or with respect to each other along the optical axis, so that the phase shifts of G1 and G2 is tuned to be $\pi$ shift or $\pi/2$ shift to match a tunable photon energy therefore the arrangement can work at different photon energy with the same phase shift gratings.

**11.** The arrangement according to any of the preceding claims, wherein the phase gratings G1 and G2 are identical, featuring a symmetric system design, that is to be said, $l_1 = l_2$ and $d_1 = d_2$.

**12.** An arrangement according to any of the preceding claims 2 to 10, wherein the phase gratings G1 and G2 have different pitches, featuring an asymmetric system design.

**13.** The arrangement according to any of the preceding claims, wherein G0 is not used but the X-ray source comprises 1D or 2D array of individual sources that may be mutually incoherent and whose lateral separation $p_{0=}p'_1 \times \dfrac{l_1}{d_1}$ or integer multiples thereof.

**14.** The arrangement according to any of the preceding claims 2 to 12, wherein the array of X-ray source is generated by using an anode that structured topographically or assembled in a mosaic manner from the same or different materials.

**15.** The arrangement according to any of the preceding claims, wherein a mechanism is comprised to place a sample (S) to be investigated between the X-ray source (or G0 if G0 is used) and G1, or between G1 and G2, or between G2 and the detector.

**16.** The arrangement according to any of the preceding claims, wherein an analysis procedure is implemented for obtain the absorption, differential phase contrast and scattering contrast of the sample that comprises the steps of recording two intensity images of the interference pattern (with sample and without sample) on the detector, and analyzing the local distortions of the two interference patterns by either spatial Fourier analysis or spatial correlation analysis, and then retaining the three contrast of the sample.

**17.** The arrangement according to any of the preceding claims, wherein a mechanism is comprised to lateral move one of the gratings (G0, G1 and G2) with a fraction of its pitch and records a serial of images on the detector, and an analysis procedure is implemented for this phase-stepping data scan that comprises the steps of calculating, for each element of the detector, the Fourier transform of the intensity curve measured in the element, and then retaining the Fourier components as signals for further processing.

**18.** The arrangement according to any of the preceding claims, comprising means for rotating the sample relatively to the remaining components to perform data collection for a tomographic scan.

**19.** The arrangement according to any of the preceding claims, wherein the phase grating G1 and G2 are fabricated on the two sides of the same wafer, with grating structures either parallel to each other or with a predefined angle.

**20.** The arrangement according to any of the preceding claims, wherein G1 is a 2D chessboard/mesh-type grating, and G2 is a 2D array grating, with each element of the array to be a circular grating.

**21.** The arrangement according to any of the preceding claims, wherein G1 and G2 are absorption gratings.

FIG 1

## FIG 2A

## FIG 2B

Virtual structured illumination (G1') generated by G1

Interference pattern (G2') on the detector plane

## FIG 2C

FIG 3A

FIG 3B

FIG 3C

FIG 3D

FIG 4

## FIG 5A

1cm

## FIG 5B

1cm

## FIG 6

Europäisches
Patentamt

European
Patent Office

Office européen
des brevets

# EUROPEAN SEARCH REPORT

Application Number

EP 15 18 3946

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/036795 A1 (ROESSL EWALD [DE] ET AL) 5 February 2015 (2015-02-05) * paragraph [0006] - paragraph [0011] * * paragraph [0084] - paragraph [0085] * * figures * | 1-21 | INV. G01N23/04 A61B6/00 G21K1/00 |
| X | EP 1 447 046 A1 (SCHERRER INST PAUL [CH]) 18 August 2004 (2004-08-18) * paragraph [0023] - paragraph [0027] * * paragraph [0033] * * paragraph [0046]; figure 2 * | 1-21 | |
| A | WO 2014/194995 A1 (SCHERRER INST PAUL [CH]) 11 December 2014 (2014-12-11) * page 7 - page 8; figures * | 1-21 | |
| A | EP 1 731 099 A1 (SCHERRER INST PAUL [CH]) 13 December 2006 (2006-12-13) * paragraph [0003] - paragraph [0005] * * paragraph [0011] - paragraph [0017] * | 1-21 | TECHNICAL FIELDS SEARCHED (IPC) G01N A61B G21K |

The present search report has been drawn up for all claims

| Place of search | Date of completion of the search | Examiner |
|---|---|---|
| The Hague | 9 February 2016 | Savage, John |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 15 18 3946

This annex lists the patent family members relating to the patent documents cited in  the above-mentioned European search report.
The members are as contained in the European Patent Office EDP file on
The European Patent Office is in no way liable for these particulars which are merely given for the purpose of information.

09-02-2016

| Patent document cited in search report | | Publication date | Patent family member(s) | | Publication date |
|---|---|---|---|---|---|
| US 2015036795 | A1 | 05-02-2015 | CN 104066375 A | | 24-09-2014 |
| | | | EP 2806798 A1 | | 03-12-2014 |
| | | | JP 2015503988 A | | 05-02-2015 |
| | | | US 2015036795 A1 | | 05-02-2015 |
| | | | WO 2013111050 A1 | | 01-08-2013 |
| EP 1447046 | A1 | 18-08-2004 | AU 2003275964 A1 | | 06-09-2004 |
| | | | EP 1447046 A1 | | 18-08-2004 |
| | | | WO 2004071298 A1 | | 26-08-2004 |
| WO 2014194995 | A1 | 11-12-2014 | NONE | | |
| EP 1731099 | A1 | 13-12-2006 | AU 2006257026 A1 | | 14-12-2006 |
| | | | CA 2610934 A1 | | 14-12-2006 |
| | | | CN 101257851 A | | 03-09-2008 |
| | | | EP 1731099 A1 | | 13-12-2006 |
| | | | EP 1887936 A1 | | 20-02-2008 |
| | | | JP 5162453 B2 | | 13-03-2013 |
| | | | JP 2008545981 A | | 18-12-2008 |
| | | | US 2009092227 A1 | | 09-04-2009 |
| | | | WO 2006131235 A1 | | 14-12-2006 |

EPO FORM P0459

For more details about this annex : see Official Journal of the European Patent Office, No. 12/82